# EUROPÄISCHE PATENTSCHRIFT

(11) **EP 0 823 973 B1**
(45) Veröffentlichungstag und Bekanntmachung des Hinweises auf die Patenterteilung: **13.09.2000**
(21) Anmeldenummer: 96909958.9
(22) Anmeldetag: 04.04.1996
(51) Int. Cl.: G01N 33/68, G01N 33/02, G01N 33/12

(54) **QUALITATIVER NACHWEIS VON ZUGESETZTEN EIWEISSHYDROLYSATEN IN LEBENSMITTELN**
QUALITITATIVE DETECTION OF ADDED ALBUMEN HYDROLYSATES IN FOODSTUFFS
IDENTIFICATION QUALITATIVE D'HYDROLYSATS D'ALBUMEN AJOUTES A DES ALIMENTS

(30) Priorität: 05.05.1995 DE 19516077
(43) Veröffentlichungstag der Anmeldung: 18.02.1998
(73) Patentinhaber: Timmermann, Detlef, 31860 Emmerthal (DE)
(72) Erfinder: Timmermann, Detlef, 31860 Emmerthal (DE)
(74) Vertreter: Rehmann, Klaus-Thorsten, Dipl.-Ing.
(86) Internationale Anmeldenummer: DE9600645
(87) Internationale Veröffentlichungsnummer: WO9635125

(56) Entgegenhaltungen:
- WO-A-87/07722
- WO-A-90/14438

## Beschreibung

Die Erfindung betrifft ein Verfahren zum qualitativen Nachweis von zugesetzten Eiweißhydrolysaten in Lebensmitteln, insbesondere von zugesetzten tierischen und pflanzlichen Eiweißhydrolysaten in Fleisch- und Fleischwaren.

Die Lebensmittelchemie ist ein Zweig der angewandten Chemie, der sich mit der Chemie der Lebensmittel, unter anderem der Ermittlung der Zusammensetzung und Funktion von Lebensmitteln und Lebensmittelzubereitungen, beschäftigt. Die Überwachung der Hersteller, des Handels und des Verkehrs mit Lebensmitteln und der Nachweis möglicher Täuschung und Irreführung des Verbrauchers sind die Schwerpunkte dieses Gebietes. Qualitative und quantitative Analysen von Lebensmitteln auf erlaubte oder verbotene Lebensmittelzusatzstoffe, auf Rückstände von Pestiziden, Düngemitteln, Lösungsmitteln, Antibiotika, Hormonen und Schwermetallen sind besondere Untersuchungsschwerpunkte.

Durch die Globalisierung des zwischenstaatlichen Handels, von dem auch die Lebensmittelindustrie in starkem Maße betroffen ist, sind zuverlässige und reproduzierbare Analysemethoden zum Schutze des Verbrauchers unerläßlich. Die Manipulation der Lebensmittel, insbesondere durch Zusatz von Stoffen, die zum Strecken der einzelnen Produkte dienen, sind in vielen Fällen verboten. Dieses betrifft insbesondere den Zusatz von tierischen und pflanzlichen Eiweißhydrolysaten in Fleisch und Fleischwaren. Häufig werden bei diesen zum Beispiel der Wassergehalt unzulässig erhöht, so daß ohne Zusatz der Eiweißhydrolysate das gesetzlich vorgeschriebene Eiweiß-Wasser-Verhältnis im nicht mehr zugelassenen Bereich liegen würde. Diese Tatsache berücksichtigend werden häufig bei Fleisch und Fleischwaren, insbesondere Hähnchenbrustfilets und Kochschinken, die nicht zugelassenen Eiweißhydrolysate zugesetzt.

Aus dem Stand der Technik ist bis heute kein zuverlässiges Analyseverfahren zur Bestimmung von zugesetzten tierischen und pflanzlichen Eiweißhydrolysaten in Lebensmitteln, insbesondere in Fleisch und Fleischwaren, bekannt. Problematisch ist vor allem die Tatsache, daß die zugesetzten Eiweißhydrolysate keine speziesfremden Stoffe darstellen, sondern zum Spektrum der natürlichen Stoffe zählen und in einer gewissen Schwankungsbreite vorkommen. Die Eiweißhydrolysate stellen Gemische von Aminosäuren, Di- und Oligo-Peptiden dar, was eine Lebensmittel-Analyse aufgrund der Vielzahl der denkbaren Verbindungen nahezu ausschließt. Dieses erschwert den Nachweis, daß einem Lebensmittel solche Eiweißhydrolysate zugesetzt worden sind, erheblich, so daß nicht nur ein quantitativer, sondern auch ein qualitativer Nachweis dieser zugesetzten Eiweißhydrolysate nahezu unmöglich erscheint.

Grundsätzlich ist es bekannt, den für Nahrungsmittel wichtigen Gehalt an Aminosäuren festzustellen. In "Journal of Chromatography" 615 (1993), Seiten 1 bis 22 ist ein Verfahren beschrieben, bei dem Nahrungsmittel zu freier Aminosäuren enthaltende Proben aufbereitet und anschließend chromatographiert werden. Ein ähnliches Verfahren ist aus WO 87/07722 bekannt, bei dem der Lysingehalt und der Gesamtproteingehalt chromatographisch bestimmt werden.

Durch "Deutsche Lebensmittel-Rundschau" 1969, Seiten 139 bis 144 ist es bekannt, einen Nachweis von Wurstwaren zugesetztem Hühnereiklar dadurch vorzunehmen, daß der Gehalt an Aminozukkern bestimmt wird. Hierzu werden die Eiweißstoffe hydrolysiert und die Aminozucker abgespalten und chromatograhisch bestimmt. Eine Bestimmung von zugesetzten Eiweißhydrolysaten ist mit diesem Verfahren weder vorgesehen noch möglich.

Das sich der Erfindung zugrundeliegende Problem ergibt sich somit darin, ein Verfahren zum qualitativen Nachweis von zugesetzten Eiweißhydrolysaten in Lebensmitteln, insbesondere von zugesetzten tierischen und pflanzlichen Eiweißhydrolysaten in Fleisch und Fleischwaren, bereitzustellen.

Dieses Problem wird erfindungsgemäß mit einem Verfahren nach Anspruch 1 gelöst.

Bei diesem Verfahren werden die Lebensmittel zu freie Aminosäuren enthaltenden Proben aufbereitet, die anschließend einer Aminosäurenbestimmung unterworfen werden, um die freien Aminosäuren und deren Gehalte zu bestimmen. Die Probenaufbereitung sowie die Aminosäurenbestimmung verlaufen nach konventionellen Methoden. Beispielsweise werden die Lebensmittel zunächst genau eingewogen, mit destilliertem Wasser verdünnt, homogenisiert und anschließend die flüssige Phase mit den darin enthaltenen freien Aminosäuren ionenaustauschchromatographisch getrennt und nach Farbreaktionen mit Ninhydrin oder o-Phthaldialdehyd nachgewiesen. Dieser Vorgang ist in der Regel automatisiert. Selbstverständlich ist es auch denkbar, daß die Aminosäuren zum Beispiel mit Phenylsenföl, FMOC, Dabsylchlorid oder Dansylchlorid derivatisiert und einer Flüssigkeitschromatographie, insbesondere einer HPLC (High Performance Liquid Chromatography) mit Reversed-Phase-Charakteristik, unterworfen werden. Die erhaltenen Chromatogramme werden sowohl qualitativ als auch quantitativ ausgewertet. Die zu den einzelnen Proben ermittelten freien Aminosäure-Mengenangaben, die aus mit Eiweißhydrolysaten behandelten Lebensmitteln stammen, werden mit solchen Proben-Mengenangaben verglichen, die aus den entsprechenden unbehandelten Lebensmitteln stammen, denen also keine Eiweißhydrolysate zugesetzt worden sind. Überschreiten einige Aminosäuregehalte die aus den entsprechenden, unbehandelten Lebensmittelproben ermittelten Werte, so ist dies zumindest ein Indiz für eine unzulässige Eiweißhydrolysat-Zugabe, jedoch noch kein Beweis, da die in Spezies vorkommenden freien Aminosäuren immer einer gewissen Schwankungsbreite unterliegen. Bei Vergleich der ermittelten Gehalte der einzelnen Aminosäuren stellt man jedoch überraschenderweise fest, daß einzelne Aminosäuregehalte trotz Zugabe von Eiweißhydrolysaten in Lebensmitteln, insbesondere in Fleisch und Fleischwaren, annähernd konstant bleiben, bei Eiweißhydrolysat-Zusatz tierischen Ursprungs vorzugsweise Serin und Asparaginsäure, während andere Aminosäuregehalte sich signifikant verändern, vorzugsweise Glycin und Alanin. Diese überraschende Erkenntnis erlaubt somit zumindest den direkten Vergleich der in einer natürlichen Schwankungsbreite vorkommenden, jedoch bei Eiweißhydrolysat-Zusatz sich signifikant verändernden Aminosäuregehalte mit aus Erfahrung festgelegten Werten, um Rückschlüsse auf eventuelle Zusätze in Form von Eiweißhydrolysaten zu ziehen bzw., erlaubt, falls ohne Eiweißhydrolyssat versetzte entsprechende Lebensmittel zur Verfügung stehen, sogar den Vergleich der ermittelten Probengehalte an bei Eiweißhydrolysat-Zugabe sich mengenmäßig signifikant verändernden freien Aminosäuren mit Probengehalten an diesen freien Aminosäuren, die aus nicht mit Eiweißhydrolysaten behandelten Lebensmitteln stammen. Durch die Signifikanz der zur Beurteilung herangezogenen Werte ist eine sichere Aussage über Eiweißhydrolysat-Zusätze möglich.

Dadurch, daß bei Eiweißhydrolysat-Zusatz tierischen Ursprungs bestimmte Aminosäuregehalte sich signifikant verändern, während andere nahezu unverändert bleiben, ist es zweckmäßig, für einen Zusatz von Eiweißhydrolysaten tierischen Ursprungs Probengehalte freier Aminosäuren, die sich durch den Zusatz erhöhen, mit Probengehalten freier Aminosäuren, die durch den Zusatz praktisch unverändert bleiben, zur Durchführung des Vergleichs in Beziehung zu setzen. Insbesondere Glycin und Alanin verändern sich bei Eiweißhydrolysatzugabe tierischen Ursprungs signifikant, während Serin und Asparaginsäure nahezu konstant bleiben. Die Quotienten werden auf die Art und Weise gebildet, daß ein bei Zusatz sich stark verändernder Aminosäuregehalt durch einen nahezu unabhängigen Aminosäuregehalt dividiert wird, wobei auch der Kehrwert eines solchen Quotienten verwendbar ist. Die Quotientenbildung ermöglicht es, Proben augenfällig als mit tierischen Eiweißhydrolysaten behandelt zu identifizieren, deren Aminosäuregehalte trotz unzulässiger Zusätze noch in der natürlichen Schwankungsbreite liegen und somit auch diese Grenzfälle sicher erfaßt werden.

Da bei Zugabe von pflanzlichen Eiweißhydrolysaten nahezu sämtliche Gehalte an freien Aminosäuren zunehmen, ist es zweckmässig, daß für einen Zusatz von pflanzlichen Eiweißhydrolysaten Probengehalte im wesentlichen alle freien Aminosäuren zur Durchführung des Vergleichs verwendet werden.

Handelt es sich bei dem zugesetzten Eiweißhydrolysat um eine Mischung von pfanzlichen und tierischen Eiweißhydrolysaten, so ist sowohl der Vergleich von Probengehalten im wesentlichen aller freien Aminosäuren als auch ein Quotientenvergleich hilfreich.

Damit die Reproduzierbarkeit der Ergebnisse des erfindungsgemäßen Verfahrens gesteigert wird, ist es zweckmäßig, daß bei der Probenaufbereitung die in den Lebensmitteln enthaltenen freien Aminosäuren von den in den Lebensmitteln enthaltenen Proteinen getrennt werden, so daß sich die Proteine nicht störend bei der Aminosäurebestimmung auswirken können.

Darüber hinaus ist es vorteilhaft, wenn die in den Lebensmitteln enthaltenen Proteine vor Trennung von den freien Aminosäuren zunächst aus den Lebensmitteln ausgefällt werden, damit die Trennung der Proteine von den freien Aminosäuren nicht durch in den Lebensmitteln enthaltenen anderen Stoffen gestört wird.

Zweckmäßigerweise wird eine einfach durchzuführende Trennung der Proteine von den Aminosäuren in der Form bewerkstelligt, daß die ausgefällten Proteine vor der Bestimmung der freien Aminosäuren von diesen abfiltriert werden. Dieser Schritt erhöht die Reproduzierbarkeit des erfindungsgemäßen Verfahrens.

Das erfindungsgemäße Nachweisverfahren ist wirtschaftlich gesehen interessant, da keine zusätzlichen speziell für das Verfahren entwickelte Apparaturen notwendig sind, so daß die sich in einem Laborpark befindenden Geräte verwendet werden können.

Das in der Zeichnung und in den Tabellen gezeigte Beispiel soll die Erfindung näher erläutern.

Es zeigen:

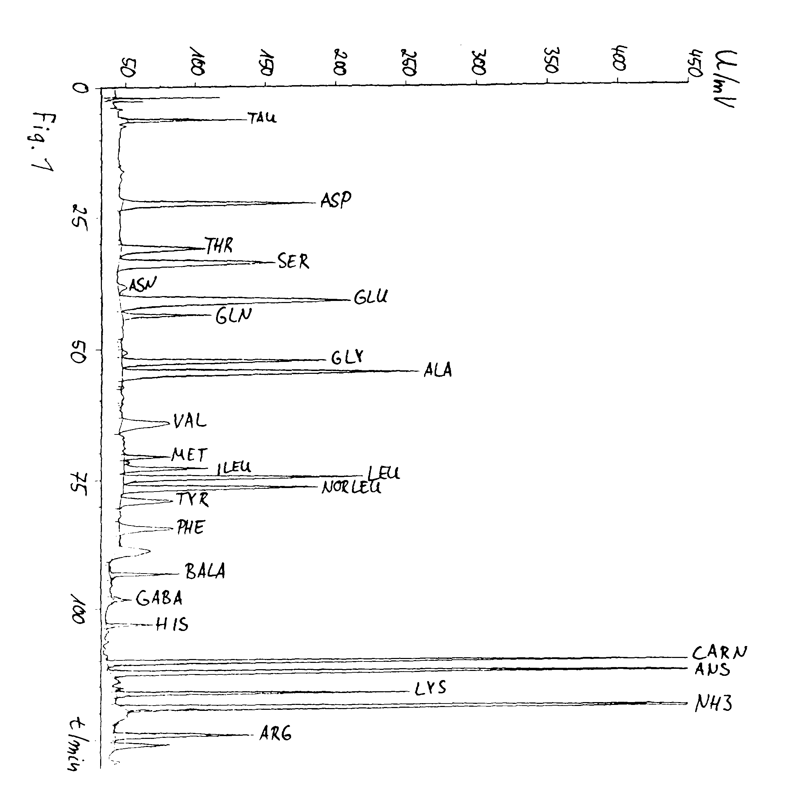

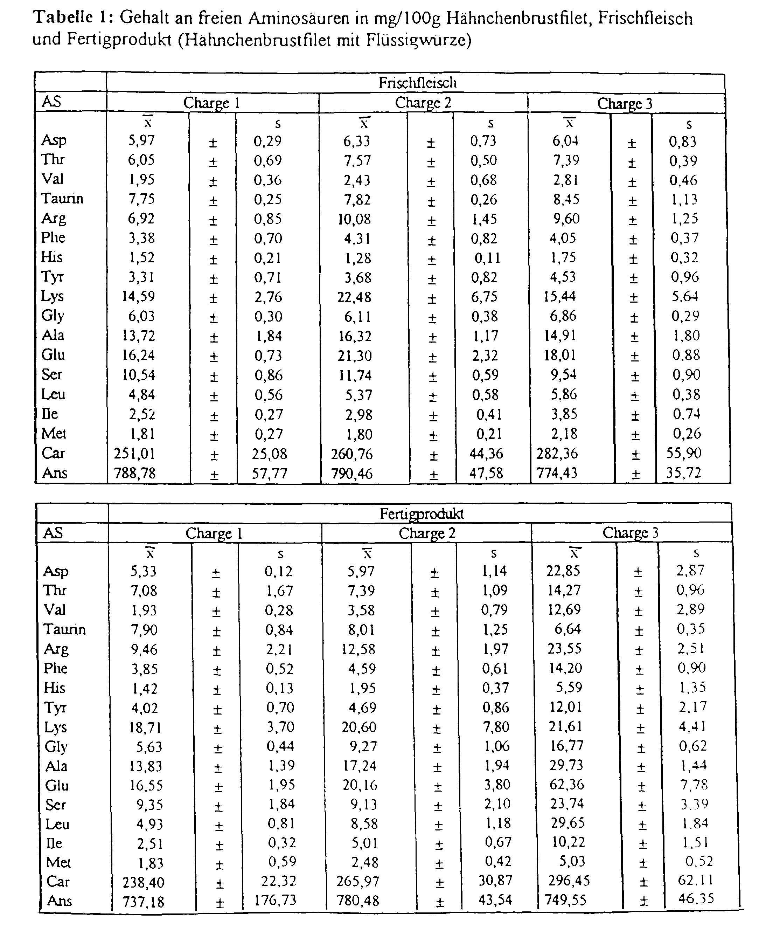

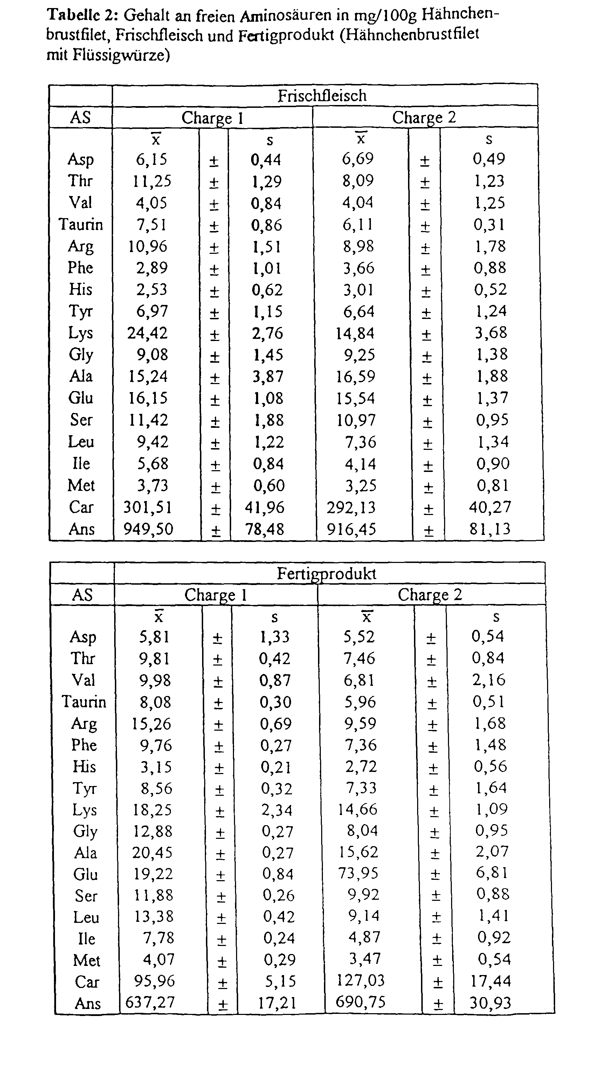

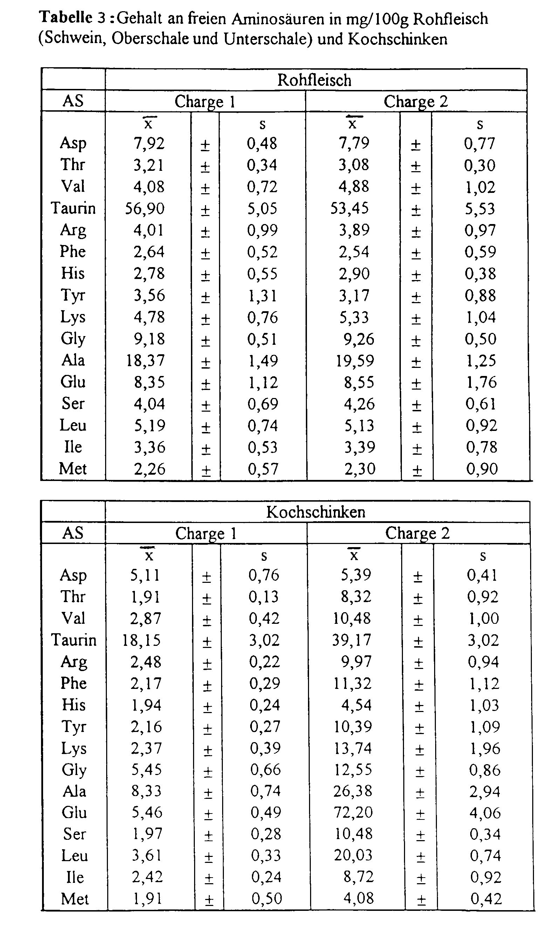

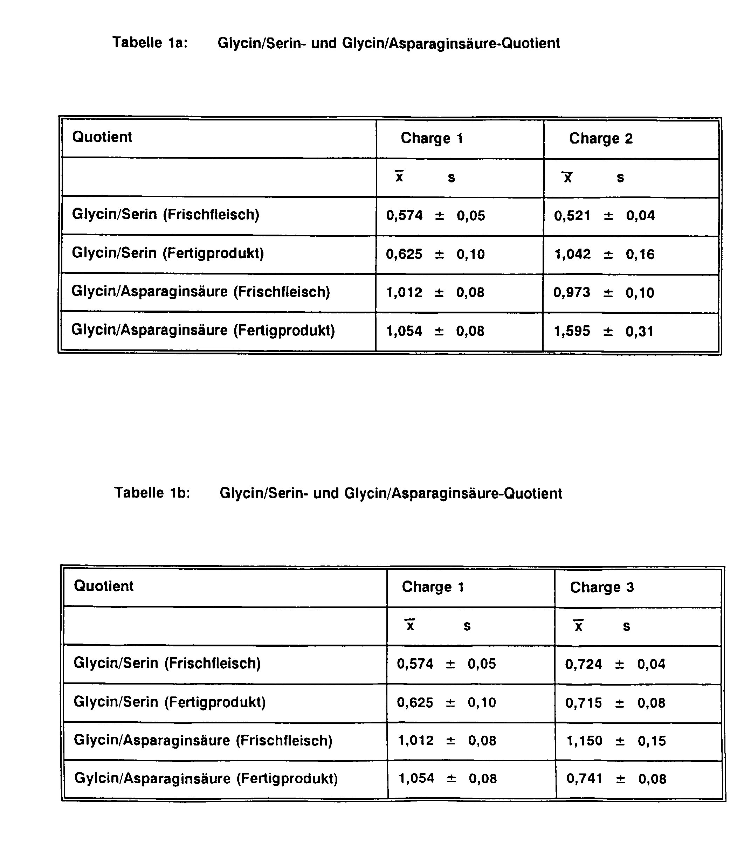

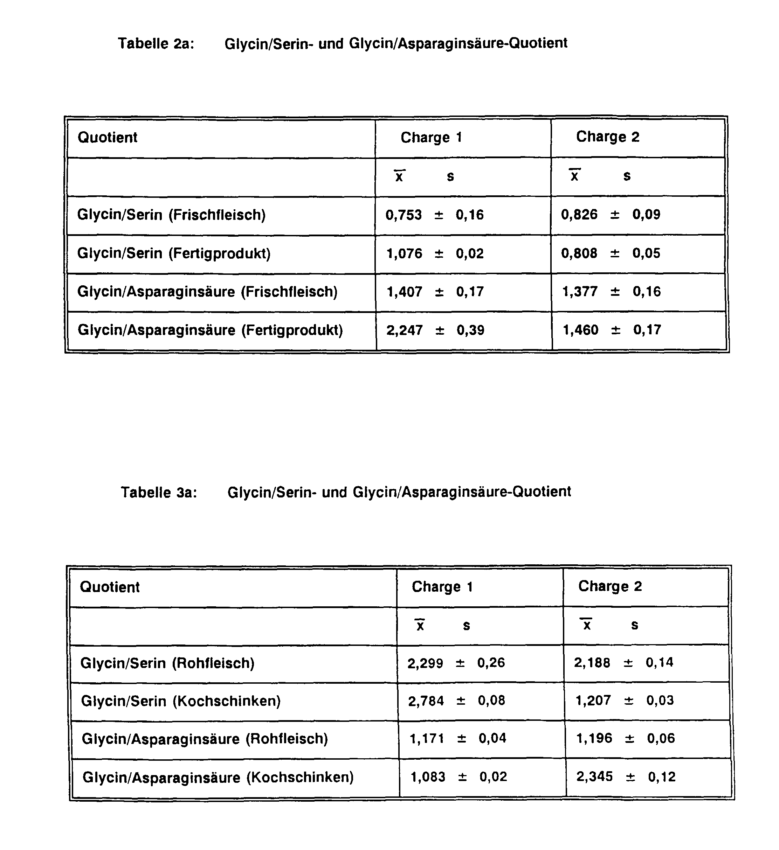

- Figur 1: - ein Aminosäurechromatogramm einer aufbereiteten Probe;
- Tabelle 1: - den Gehalt an freien Aminosäuren in mg/100g Hähnchenbrustfilet von Frischfleisch ohne Eiweißhydrolysat-Zusatz und einem Fertigprodukt jeweils ohne, mit tierischem und mit pflanzlichem Eiweißhydrolysatzusatz;
- Tabelle 2: - den Gehalt an freien Aminosäuren in mg/100g Hähnchenbrustfilet von Frischfleisch ohne Eiweißhydrolysat-Zusatz und einem Fertigprodukt jeweils ohne und mit tierischem Eiweißhydrolysatzusatz;
- Tabelle 1a: - Glycin/Serin- und Glycin/Asparaginsäure-Quotienten, die mit Werten der Chargen 1 und 2 aus Tabelle 1 gebildet worden sind;
- Tabelle 1b: - Glycin/Serin- und Glycin/Asparaginsäure-Quotienten, die mit Werten der Chargen 1 und 3 aus Tabelle 1 gebildet worden sind;
- Tabelle 2a: - Glycin/Serin- und Glycin/Asparaginsäure-Quotienten, die mit Werten aus Tabelle 2 gebildet worden sind;
- Tabelle 3: - den Gehalt an freien Aminosäuren in mg/100 g Rohfleisch (ohne Eiweißhydrolysatzusatz) vom Schwein (Ober- und Unterschale) und Kochschinken jeweils ohne und mit Eiweißhydrolysatzusatz und
- Tabelle 3a: - Glycin/Serin- und Glycin/Asparagin-Quotienten, die mit Werten aus Tabelle 3 gebildet worden sind.

Figur 1 zeigt ein Aminosäurechromatogramm einer mit Eiweißhydrolysat versetzten aufbereiteten Probe, in dem den einzelnen Peaks die jeweilige Aminosäure zugeordnet ist. Die einzelnen Peakflächen entsprechen den jeweiligen Aminosäuremengen. Diese werden direkt mit aus ohne Eiweißhydrolysat behandelten Proben erhaltenen Werten bzw. über Quotienten verglichen, die in der Form gebildet werden, daß ein bei Zusatz von Eiweißhydrolysaten sich stark verändernder Aminosäuregehalt durch einen nahezu unabhängigen Aminosäuregehalt dividiert wird, so daß Aussagen darüber möglich sind, ob eine Lebensmittelprobe mit Eiweißhydrolysaten versetzt worden ist oder nicht.

Die in den Tabellen angegebenen Mittelwerte, Quotienten und deren Varianzen wurden jeweils mit n=6 Werten mit Hilfe der zweifaktoriellen Varianzanalyse bestimmt.

Zunächst ist anzumerken, daß die in den nachfolgenden Tabellen aufgeführten Frischfleisch-/Rohfleisch-Chargen nicht mit Eiweißhydrolysat versetzt worden sind. Die zu diesen Chargen angegebenen Werte zeigen eine natürliche Schwankungsbreite der einzelnen Aminosäuregehalte und dienen als Vergleichsparameter für eine Bewertung hinsichtlich eines Eiweißhydrolysatzusatzes.

Aus der Tabelle 1 ist zu erkennen, daß bei Eiweißhydrolysat-zusatz zu einem Fertigprodukt (Chargen 2 und 3) eine signifikante Veränderung der freien Aminosäuren stattgefunden hat. Bei Zugabe von tierischen Eiweißhydrolysaten zum Fertigprodukt (Charge 2) sind nur bestimmte Aminosäuregehalte signifikant verändert, in den meisten Fällen erhöht, während bei Zugabe von pflanzlichen Eiweißhydrolysaten (Charge 3) nahezu sämtliche Aminosäuregehalte erheblich zugenommen haben. Die Mittelwertvergleiche zeigen, daß die Aminosäuren Glycin, Alanin, Leucin und Isoleucin im Fertigprodukt von der Charge 2 (mit tierischem Eiweißhydrolysatzusatz) und die Aminosäuren Asparaginsäure, Threonin, Valin, Arginin, Phenylalanin, Histidin, Tyrosin, Glycin, Alanin, Glutaminsäure, Serin, Leucin, Isoleucin und Methionin der Charge 3 (mit pflanzlichem Eiweißhydrolysatzusatz) im Vergleich zur Charge 1 (ohne Eiweißhydrolysatzusatz) einen signifikant erhöhten Gehalt aufweisen.

Eine Zugabe von tierischem Eiweißhydrolysat erhöht den Glycingehalt von der in der Tabelle 1 gezeigten Charge 2 (Fertigprodukt) und der in der Tabelle 2 gezeigten Charge 1 (Fertigprodukt), wobei gleichzeitig der Gehalt der Aminosäuren Serin und Asparaginsäure nahezu konstant bleibt. Aufgrund dieser Tatsache werden als zusätzliches Bewertungskriterium die Glycin/-Serin- und Glycin/Asparaginsäurequotienten verwendet, die in der Tabelle 1a gezeigt sind.

In der Tabelle 1a ist zu erkennen, daß der Glycin/Serin-Quotient vom Fertigprodukt der Charge 2 (mit tierischem Eiweißhydrolysatzusatz) im Vergleich zu den entsprechenden Quotienten des Frischfleisches der Chargen 1 und 2 und des Fertigproduktes der Charge 1 (alle ohne Eiweißhydrolysatzusatz) signifikant höher liegt. Das gleiche gilt für den Glycin/Asparaginsäure-Quotienten.

In der Tabelle 1b ist zu erkennen, daß der Glycin/Serin-Quotient vom Fertigprodukt der Charge 3 (mit pflanzlichem Eiweißhydrolysatzusatz) im Vergleich zu den entsprechenden Quotienten des Frischfleisches der Chargen 1 und 3 und des Fertigproduktes der Charge 1 (alle ohne Eiweißhydrolysatzusatz) in der gleichen Größenordnung liegt. Beim Glycin/Asparaginsäure-Quotienten vom Fertigprodukt der Charge 3 liegt dieser sogar deutlich unter den entsprechenden Quotienten des Frischfleisches der Chargen 1 und 3 und des Fertigproduktes der Charge 1. Dies deutet daraufhin, daß dem Fertigprodukt der Charge 3 zumindest kein tierisches Eiweißhydrolysat zugesetzt worden ist. Über den oben genannten Mittelwertvergleich der einzelnen Aminosäuren zur Charge 1 des Fertigproduktes ist eine sichere Aussage darüber möglich, daß ein pflanzlicher Eiweißhydrolysatzusatz zum Fertigprodukt der Charge 3 gegeben ist.

In der Tabelle 2 ist zu erkennen, daß die Aminosäuren Arginin, Glycin, Alanin, Leucin und Isoleucin im Fertigprodukt der Charge 1 (mit tierischem Eiweißhydrolysatzusatz) im Vergleich zu Charge 2 (ohne Eiweißhydrolysatzusatz) signifikant höher liegen. Der höhere Gehalt an Glutaminsäure im Fertigprodukt der Charge 2 gegenüber Charge 1 beruht auf der Zugabe von Glutamat, das als Geschmacksverstärker in vielen Fertigprodukten Verwendung findet.

Tabelle 2a zeigt, daß bei den in der Tabelle 2 angegebenen Werten der Glycin/Serin-Quotient vom Fertigprodukt der Charge 1 (mit tierischem Eiweißhydrolysatzusatz) im Vergleich zum Frischfleisch der Chargen 1 und 2 und zum Fertigprodukt der Charge 2 (alle ohne Eiweißhydrolysatzusatz) signifikant höher liegt. Das gleiche gilt für den Glycin/Asparaginsäure-Quotienten.

Da sich die Alanin-Gehalte ähnlich verhalten wie die Glycin-Gehalte, können als weitere Bewertungskriterien die Quotienten Alanin/Serin und Alanin/Asparaginsäure eingesetzt werden.

Die Quotienten, insbesondere die Quotienten Glycin/Serin und Glycin/Asparaginsäure, haben eine besondere Bedeutung bei der Bewertung von Grenzfällen, das heißt von Fällen, bei denen trotz Eiweißhydrolysat-Zusatzes die aus den entsprechenden, nicht mit Eiweißhydrolysaten behandelten Lebensmitteln ermittelten einzelnen Aminosäuregehalte nicht überschritten werden. Diese Grenzfälle sind darauf zurückzuführen, daß eine natürliche Schwankungsbreite der einzelnen Aminosäuregehalte vorhanden ist. Beispielsweise liegt der in Tabelle 1 gezeigte Glycingehalt beim Fertigprodukt der Charge 2 (mit tierischem Eiweißhydrolysat) mit 9,27 mg/100 g Gesamtmasse auf der Höhe des in der Tabelle 2 gezeigten Glycin-Gehaltes des Frischfleisches der Charge 2 (ohne Eiweißhydrolysatzusatz) mit 9,25 mg/100 g Gesamtmasse. Eine sichere Beurteilung, ob dem Fertigprodukt Eiweißhydrolysate zugesetzt worden sind, ist somit erschwert bzw. nicht möglich. Dagegen ist der in der Tabelle 1a gezeigte Glycin/Serin-Quotient vom Fertigprodukt der Charge 2 (mit tierischem Eiweißhydrolysatzusatz) mit 1,042 signifikant höher als der in der Tabelle 2a gezeigte Glycin/Serin-Quotient vom Frischfleisch der Charge 2 (ohne Eiweißhydrolysatzusatz) mit 0,826. Der Vergleich der beiden Quotienten zeigt deutlich, daß dem Fertigprodukt tierische Eiweißhydrolysate zugesetzt worden sind.

In Tabelle 3 ist zu erkennen, daß ein Eiweißhydrolysatzusatz (Charge 2) bei der Herstellung von Kochschinken zu einer signifikanten Veränderung der freien Aminosäuren führt. Die anschließenden Mittelwertsvergleiche zeigen, daß die Aminosäuren Threonin, Valin, Arginin, Phenylalanin, Histidin, Tyrosin, Lysin, Glycin, Alanin, Glutaminsäure, Serin, Leucin, Isoleucin und Methionin im Fertigprodukt von der Charge 2 (mit Eiweißhydrolysatzusatz) im Vergleich zur Charge 1 (ohne Eiweißhydrolysatzusatz) einen signifikant erhöhten Gehalt aufweisen. Dies zeigt, daß dem Kochschinken der Charge 2 zumindest pflanzliche Eiweißhydrolysate zugesetzt worden sind. Ist es ferner von Interesse, in Erfahrung zu bringen, ob dem Kochschinken der Charge 2 auch tierische Eiweißhydrolysate zugesetzt worden sind, kann ein entsprechender Vergleich der oben genannten Quotienten herangezogen werden.

Die in Tabelle 3a gezeigten Glycin/Serin- bzw. Glycin/Asparaginsäure-Quotienten, die aus den in der Tabelle 3 angegebenen Werten berechnet werden, deuten auf einen tierischen Eiweiß hydrolysatzusatz hin. Es ist zu erkennen, daß der Glycin/Serin-Quotient des Kochschinkens aus Charge 2 (mit Eiweißhydrolysatzusatz) deutlich kleiner als die Glycin/Serin-Quotienten des Rohfleisches der beiden Chargen 1 und 2 und des Kochschinkens der Charge 1 (alle ohne Eiweißhydrolysatzusatz) ist. Dies läßt keine Aussage darüber zu, ob dem Kochschinken aus Charge 2 tierische Eiweißhydrolysate zugesetzt worden sind. Jedoch stellt sich bei Vergleich des Glycin/Asparaginsäure-Quotienten des Kochschinkens aus Charge 2 (mit Eiweißhydrolysatzusatz) heraus, daß dieser erheblich über den entsprechenden Glycin/Asparaginsäure-Quotienten des Rohfleisches der beiden Chargen 1 und 2 bzw. des Kochschinkens aus Charge 1 (alle ohne Eiweißhydrolysatzusatz) liegt. Dies ist ein deutlicher Hinweis darauf, daß dem Kochschinken aus Charge 2 neben den pflanzlichen Eiweißhydrolysaten zusätzlich noch tierische Eiweißhydrolysate zugesetzt worden sind. Die Tatsache, daß der Glycin/Serin-Quotient diese Aussage nicht stützt, kann dahingehend gedeutet werden, daß der pflanzliche Eiweißhydrolysatzusatz einen relativ hohen Serin-Anteil aufweist, so daß der entsprechende Glycin/Serin-Quotient erheblich verkleinert und somit die Anwesenheit tierischen Eiweißhydrolysates quasi "verdeckt" wird.

## Patentansprüche

1. Verfahren zum qualitativen Nachweis von zugesetzten Eiweißhydrolysaten in Lebensmitteln, insbesondere von zugesetzten tierischen und pflanzlichen Eiweißhydrolysaten in Fleisch und Fleischwaren, **dadurch gekennzeichnet, daß**
- die Lebensmittel zu freie Aminosäuren enthaltenden Proben aufbereitet werden und
- die in den aufbereiteten Proben vorhandenen freien Aminosäuren qualitativ und quantitativ bestimmt werden, wobei die ermittelten Probengehalte an freien Aminosäuren mit solchen Probengehalten verglichen werden, die aus den entsprechenden, nicht mit Eiweißhydrolysaten behandelten Lebensmitteln stammen.

2. Verfahren nach Anspruch 1, **dadurch gekennzeichnet, daß** für einen Zusatz von Eiweißhydrolysaten tierischen Ursprungs Probengehalte freier Aminosäuren, die sich durch den Zusatz erhöhen, mit Probengehalten freier Aminosäuren, die durch den Zusatz praktisch unverändert bleiben, zur Durchführung des Vergleichs in Beziehung gesetzt werden.

3. Verfahren nach Anspruch 1, **dadurch gekennzeichnet, daß** für einen Zusatz von pflanzlichen Eiweißhydrolysaten Probengehalte im wesentlichen aller freien Aminosäuren zur Durchführung des Vergleichs verwendet werden.

4. Verfahren nach Anspruch 1, 2 oder 3, **dadurch gekennzeichnet, daß** bei der Probenaufbereitung die in den Lebensmitteln enthaltenen freien Aminosäuren von den in den Lebensmitteln enthaltenen Proteinen getrennt werden.

5. Verfahren nach Anspruch 4, **dadurch gekennzeichnet, daß** die in den Lebensmitteln enthaltenen Proteine vor Trennung von den freien Aminosäuren zunächst aus den Lebensmitteln ausgefällt werden.

6. Verfahren nach Anspruch 5, **dadurch gekennzeichnet, daß** die ausgefällten Proteine vor der Bestimmung der freien Aminosäuren von diesen abfiltriert werden.

7. Verfahren nach Anspruch 6, **dadurch gekennzeichnet, daß** vor dem Filtrieren die Proteine und die freien Aminosäuren einer Zentrifugierung unterworfen werden.

## Claims

1. Process for the qualitative detection of added protein hydrolysates in foodstuffs, in particular of added animal and vegetable protein hydrolysates in meat and meat products, characterised in that
- the foodstuffs are prepared to yield samples containing free amino acids and
- the free amino acids present in the prepared samples are qualitatively and quantitatively determined, wherein the determined sample contents of free amino acids are compared with such sample contents originating from corresponding foodstuffs which have not been treated with protein hydrolysates.

2. Process according to claim 1, characterised in that, in the case of addition of protein hydrolysates of animal origin, the comparison is performed by relating sample contents of free amino acids which are increased by the addition to sample contents of free amino acids which remain virtually unchanged by the addition.

3. Process according to claim 1, characterised in that, in the case of addition of protein hydrolysates of vegetable origin, the comparison is performed by using the sample contents of substantially all the free amino acids.

4. Process according to claim 1, 2 or 3, characterised in that, during sample preparation, the free amino acids contained in the foodstuffs are separated from the proteins contained in the foodstuffs.

5. Process according to claim 4, characterised in that, prior to separation from the free amino acids, the proteins contained in the foodstuffs are first precipitated from the foodstuffs.

6. Process according to claim 5, characterised in that, prior to determination of the free amino acids, the precipitated proteins are separated therefrom by filtration.

7. Process according to claim 6, characterised in that, prior to filtration, the proteins and the free amino acids are subjected to centrifugation.

## Revendications

1. Procédé pour la détection qualitative d'hydrolysats d'albumen dans des aliments, en particulier d'hydrolysats d'albumen animaux et végétaux ajoutés dans la viande et les denrées carnées, caractérisé en ce que :
- les aliments sont préparés en échantillons contenant des acides aminés libres et
- les acides aminés libres présents dans les échantillons préparés sont déterminés qualitativement et quantitativement, les teneurs en acides aminés libres mesurées dans les échantillons étant comparées avec des teneurs en échantillon qui proviennent des aliments correspondants non-traités avec des hydrolysats d'albumen.

2. Procédé selon la revendication 1, caractérisé en ce que pour une addition d'hydrolisats d'albumen d'origine animale, des teneurs en acides aminés libres en échantillon, qui s'élèvent par l'addition, sont mises en relation avec des teneurs en acides aminés libres en échantillon qui restent pratiquement inchangées par l'addition, pour la mise en oeuvre de la comparaison.

3. Procédé selon la revendication 1, caractérisé en ce que pour une addition d'hydrolysats d'albumen végétaux on utilise des teneurs en échantillon de sensiblement tous les acides aminés libres pour la mise en oeuvre de la comparaison.

4. Procédé selon la revendication 1, 2 ou 3, caractérisé en ce que lors de la préparation des échantillons, les acides aminés libres contenus dans les aliments sont séparés des protéines contenues dans les aliments.

5. Procédé selon la revendication 4, caractérisé en ce qu'avant leur séparation des acides aminés libres, les protéines contenues dans les aliments sont d'abord précipitées hors des aliments.

6. Procédé selon la revendication 5, caractérisé en ce qu'avant la détermination des acides aminés, les protéines précipitées en sont séparées par filtration.

7. Procédé selon la revendication 6, caractérisé en ce qu'avant la filtration, les protéines et les acides aminés libres sont soumis à une centrifugation.
